(19) Europäisches Patentamt
European Patent Office
Office européen des brevets

(11) Veröffentlichungsnummer: **0 546 449 A2**

# (12) EUROPÄISCHE PATENTANMELDUNG

(21) Anmeldenummer: **92120651.2**

(22) Anmeldetag: **03.12.92**

(51) Int. Cl.5: **C07D 471/04**, A61K 31/435, //(C07D471/04,235:00,221:00)

(30) Priorität: **09.12.91 DE 4140519**

(43) Veröffentlichungstag der Anmeldung: **16.06.93 Patentblatt 93/24**

(84) Benannte Vertragsstaaten: **AT BE CH DE DK ES FR GB GR IE IT LI LU NL PT SE**

(71) Anmelder: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Mederski, Werner, Dr.**
**Am Ohlenberg 29**
**W-6106 Erzhausen(DE)**
Erfinder: **Juraszyk, Horst, Dr.**
**Kleiner Ring 14**
**W-6104 Seeheim(DE)**
Erfinder: **Beier, Norbert Dr.**
**Georgenhäuser Strasse 19**
**W-6107 Reinheim(DE)**
Erfinder: **Schelling, Pierre, Prof. Dr.**
**Bardenbergweg 17**
**W-6109 Mühltal(DE)**
Erfinder: **Lues, Ingeborg, Dr.**
**Katharinen-Strasse 2**
**W-6100 Darmstadt(DE)**
Erfinder: **Minck, Klaus-Otto, Dr.**
**Büchestrasse 8**
**W-6105 Ober-Ramstadt(DE)**

(54) **Benzofuranderivate als Angiotensin II Antagonisten.**

(57) Neue Benzofurane der Formel I

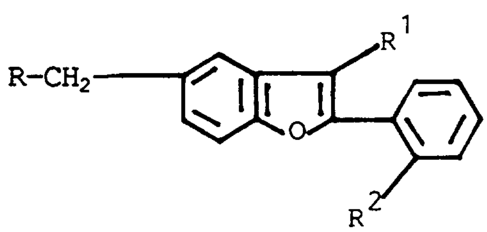

I

worin
R den Rest

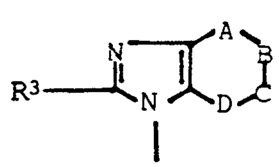

bedeutet und
$R^1$ bis $R^3$ sowie
-A-B-C-D- die in Anspruch 1 angegebene Bedeutung haben,

EP 0 546 449 A2

sowie deren Salze zeigen angiotensin II-antagonistische Eigenschaften und können zur Behandlung von Hypertension, Aldosteronismus und Herzinsuffizienz verwendet werden.

Die Erfindung betrifft neue Benzofurane der Formel I

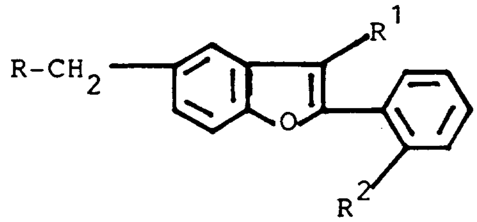

I

worin

R den Rest

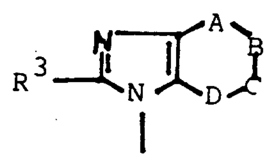

| | |
|---|---|
| $R^1$ | H, Hal, COOH, $CONH_2$, CHO, CN, $NH_2$ oder 5-Tetrazolyl, |
| $R^2$ | H, COOH, $COOR^3$, CN, $NO_2$, $NH_2$, $NHCOCF_3$, $NHSO_2CF_3$ oder 5-Tetrazolyl, |
| $R^3$ | H, Alkyl, Alkenyl oder Alkinyl mit jeweils bis zu 6 C-Atomen, |
| $R^4$ | H, Alkyl mit 1-6 C-Atomen, Cyan-alkyl, $R^3$OOC-alkyl, 5-Tetrazolyl-alkyl oder Ar-alkyl mit jeweils 1-6 C-Atomen im "alkyl"-Teil, |
| -A-B-C-D- | eine der Gruppen -CH = CH-CH = N-, -CH = CH-N = CH-, -CH = N-CH = CH-, -N = CH-CH = CH-, -CH = CH-CO-$NR^4$-, -CH = CH-$NR^4$-CO-, -CO-$NR^4$-CH = CH- oder -$NR^4$-CO-CH = CH-, worin die H-Atome der -CH-Gruppen durch Alkyl mit 1-6 C-Atomen, Hal, $COOR^3$, CN und/oder 5-Tetrazolyl substituiert sein können, |
| Ar | unsubstituiertes oder ein- oder zweifach durch Hal, $R^3$, $CF_3$, $COOR^3$, CN, $OR^3$, $NO_2$, $NH_2$, $NHCOCF_3$, $NHSO_2CF_3$ oder 5-Tetrazolyl substituiertes Phenyl und |
| Hal | F, Cl, Br oder I bedeuten, |

sowie ihre Salze.

Ähnliche Verbindungen sind aus der EP-A2-0430 709 bekannt, sie enthalten jedoch einen Benzothiophen-Ring an Stelle des Benzofuran-Rings und eine gegebenenfalls substituierte Imidazolylgruppe an Stelle des Restes R.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der angiotensin II-abhängigen Hypertension, des Aldosteronismus und der Herzinsuffizienz sowie von Störungen des Zentralnervensystems eingesetzt werden. Diese Wirkungen können nach üblichen in vitro- oder in vivo-Methoden ermittelt werden, wie sie z.B. in der US-PS 4 880 804 und in der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie, Herzinsuffizienz und Hyperaldosteronismus,
ferner von Hypertrophie und Hyperplasie der Blutgefäße und des Herzens, Angina pectoris, Herzinfarkten, Schlaganfall, Ristenosen nach Angioplastie oder By-pass-Operation, Arteriosklerose, erhöhtem Augeninnendruck, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorgaren, Wahrnehmungsstörungen, z.B. Demenz, Amnesie, Gedächtnisfunktionsstörungen, Angstzuständen, Depression und/oder Epilepsie.

Gegenstand der Erfindung sind die Verbindungen der Formel I und ihre Salze sowie ein Verfahren zur Herstellung dieser Verbindungen sowie ihrer Salze, dadurch gekennzeichnet, daß man
eine Verbindung der Formel II

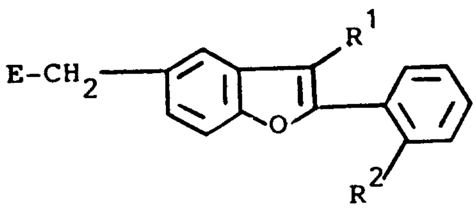

II

worin

E Cl, Br, I oder eine freie oder reaktionsfähig funktionell abgewandelte OH-Gruppe bedeutet und

$R^1$ und $R^2$ die in Anspruch 1 angegebene Bedeutung haben,

mit einer Verbindung der Formel III

H-R III

worin

R die in Anspruch 1 angegebene Bedeutung hat,

umsetzt,

oder daß man eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) $R^1$, $R^2$ und/oder R in einen oder mehrere andere Reste $R^1$, $R^2$ und/oder R umwandelt und/oder eine Base oder Säure der Formel I in eines ihrer Salze überführt.

Vor- und nachstehend haben die Reste bzw. Parameter R, $R^1$ bis $R^4$, -A-B-C-D-, Ar, Hal und E die bei den Formeln I und II angegebenen Bedeutungen, falls nicht ausdrücklich etwas anderes angegeben ist.

In den vorstehenden Formeln hat "Alkyl" 1-6, vorzugsweise 1, 2, 3, oder 4 C-Atome. "Alkyl" bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl.

Hal bedeutet vorzugsweise F, Cl oder Br, aber auch J.

R ist ein von 1H- oder 3H-Imidazo[4,5-b]pyridin oder ein von 1H- oder 3H-Imidazo[4,5-c]pyridin abgeleiteter Rest, genauer:

(a) 2-$R^3$-3H-imidazo[4,5-b]pyridin-3yl (falls -A-B-C-D- = -CH=CH-CH=N-),
(b) 2-$R^3$-3H-imidazo[4,5-c]pyridin-3-yl (falls -A-B-C-D- = -CH=CH-N=CH-),
(c) 2-$R^3$-1H-imidazo[4,5-c]pyridin-1-yl (falls -A-B-C-D- = -CH=N-CH=CH-),
(d) 2-$R^3$-1H-imidazo[4,5-b]pyridin-1-yl (falls -A-B-C-D- = -N=CH-CH=CH-),
(e) 2-$R^3$-4-$R^4$-4,5-dihydro-5-oxo-3H-imidazo[4,5-b]pyridin-3-yl (falls -A-B-C-D- = -CH-CH-CO-$NR^4$-),
(f) 2-$R^3$-5-$R^4$-4,5-dihydro-4-oxo-3H-imidazo[4,5-c]pyridin-3-yl (falls -A-B-C-D- = CH-CH-$NR^4$-CO-),
(g) 2-$R^3$-5-$R^4$-4,5-dihydro-4-oxo-1H-imidazo[4,5-c]pyridin-3-yl (falls -A-B-C-D- = -CO-$NR^4$-CH=CH-),
(h) 2-$R^3$-4-$R^4$-4,5-dihydro-5-oxo-1H-imidazo[4,5-b]pyridin-3-yl (falls -A-B-C-D- = -$NR^4$-CO-CH=CH-).

Dabei können in den Resten -A-B-C-D- die an den C-Atomen befindlichen H-Atome durch Alkyl (vorzugsweise Methyl), Hal (vorzugsweise F oder Cl), $COOR^3$ (vorzugsweise COOH, $COOCH_3$ oder $COOC_2H_5$), CN und/oder 5-Tetrazolyl substituiert sein. Als Substituenten sind bevorzugt $CH_3$ und COOH. Vorzugsweise sind nur eines oder zwei dieser H-Atome durch einen der angegebenen Substituenten substituiert.

Dementsprechend umschließen die Verbindungen der Formel I solche der Formel Ia bis Ih, worin R die jeweils unter (a) bis (h) angegebene Bedeutung hat. Die Verbindungen der Formeln Ia und If sind bevorzugt.

Der Rest $R^1$ ist vorzugsweise H oder Br.

Der Rest $R^2$ ist vorzugsweise CN, ferner bevorzugt 5-Tetrazolyl, COOH, $COOCH_3$, $COOC_2H_5$ oder $NHSO_2CF_3$.

Der Rest $R^3$ (sofern er nicht H bedeutet) ist vorzugsweise geradkettig und steht bevorzugt für Alkyl oder Alkenyl mit jeweils 2-6 C-Atomen, insbesondere Butyl, ferner Ethyl oder Propyl, weiterhin Pentyl, Hexyl, Allyl oder 1-Propenyl, ferner 1-Butenyl, 1-Pentenyl, 1-Hexenyl, Ethinyl, 1-Propinyl, 1-Butinyl, 1-Pentinyl oder

1-Hexinyl.

Der Rest $R^4$ ist vorzugsweise H, ferner bevorzugt Alkyl (insbesondere $CH_3$), Cyanalkyl (insbesondere Cyanmethyl, 2-Cyanethyl, 3-Cyanpropyl), AOOC-alkyl (insbesondere Methoxycarbonylmethyl, Ethoxycarbonylmethyl, 2-Methoxycarbonyl-ethyl, 2-Ethoxycarbonyl-ethyl), Carboxyalkyl (insbesondere Carboxymethyl, 2-Carboxyethyl, 3-Carboxypropyl), 5-Tetrazolyl-alkyl [insbesondere 5-Tetrazolyl-methyl, 2-(5-Tetrazolyl)-ethyl, 3-(5-Tetrazolyl)-propyl), wobei alle diese Reste vorzugsweise jeweils insgesamt bis zu 6 C-Atome enthalten können. Außerdem ist der Rest $R^4$ vorzugsweise Ar-alkyl mit 7-11 C-Atomen, insbesondere Benzyl, 1- oder 2-Phenylethyl, 1-, 2- oder 3-Phenylpropyl, 1-, 2-, 3- oder 4-Phenylbutyl, o-, m- oder p-Fluorbenzyl, (bevorzugt) o-, m- oder p-Chlorbenzyl, o-, m-oder p-Brombenzyl, o-, m- oder p-Methylbenzyl, o-, m- oder p-Trifluormethylbenzyl, o-, m- oder p-Methoxycarbonylbenzyl, o-, m- oder p-Ethoxycarbonylbenzyl, (bevorzugt) o-, m- oder p-Cyanbenzyl, o-, m- oder p-Carboxybenzyl, o-, m- oder p-Nitrobenzyl, (bevorzugt) o-, m- oder p-Aminobenzyl, (bevorzugt) o-, m- oder p-(5-Tetrazolyl)-benzyl.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ii, Ij, Iai bis Ihi und Iaj bis Ihj ausgedrückt werden, die den Formeln I sowie Ia bis Ih entsprechen und worin die nicht näher bezeichneten Reste die bei den Formeln I sowie Ia bis Ih angegebenen Bedeutungen haben:
Verbindungen der Formel Ii sowie Iai, Ibi, Ici, Idi, Iei, Ifi, Igi und Ihi: diese entsprechen den Formeln I sowie Ia bis Ih, zusätzlich bedeutet in ihnen jedoch $R^1$ H.

Verbindungen der Formeln Ij sowie Iaj, Ibj, Icj, Idj, Iej, Ifj, Igj, Ihj, Iij, Iaij, Ibij, Icij, Idij, Ieij, Ifij, Igij und Ihij: diese entsprechen den Formeln I sowie Ia bis Ii und Iai bis Ihi, zusätzlich bedeutet in ihnen jedoch $R^2$ CN oder (bevorzugt) 5-Tetrazolyl.

Eine weitere ausgewählte Gruppe bevorzugter Verbindungen entspricht der Formel I, worin $R^1$ H, $R^2$ 5-Tetrazolyl, $R^3$ Alkyl mit 2-4 C-Atomen und -A-B-C-D- $-C(CH_3)=CH-CH=N-$,$-C(CH_3)=CH-C(CH_3)=N-$, $-CH=CH-N(O-HOOC-C_6H_4-CH_2)-CO-$, $-CH=CH-NH-CO-$oder $-CH=CH-N(CH_2COOH)-CO-$ bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-0 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie $CH_3ONa$ oder K-tert.-Butylat in einem Alkohol wie $CH_3OH$ oder mit einem Alkalimetallhydrid wie NaH oder einem Alkalimetallalkoholat in Dimethylformamid (DMF), und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetallcarbonate wie $Na_2CO_3$ oder $K_2CO_3$ oder Alkalimetallhydrogencarbonate wie $NaHCO_3$ oder $KHCO_3$.

Die Ausgangsstoffe, insbesondere diejenigen der Formel III, sind teilweise bekannt. Falls sie nicht bekannt sind, können sie nach bekannten Methoden in Analogie zu bekannten Stoffen hergestellt werden.

Verbindungen der Formel II sind neu. Solche der Formel II (E = Br) sind z.B. erhältlich durch Reaktion von 5-Methylsalicylaldehyd mit einem $\alpha$-$R^1$-2-$R^2$-benzylbromid der Formel IV zu einem $\alpha$-$R^1$-2-$R^2$-benzylether der Formel V, Cyclisierung

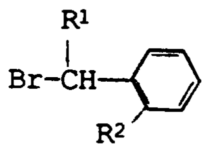 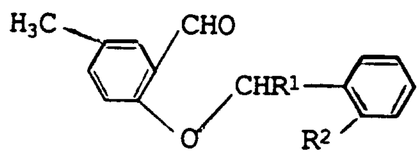

IV V

unter Wasserabspaltung (z.B. mit NaH/DMF) zum entsprechenden 3-$R^1$-2-(2-$R^2$-phenyl)-5-methylbenzofuran (Formel II, aber H an Stelle von E) und Bromierung (z.B. mit N-Bromsuccinimid).

Man kann ferner eine Verbindung der Formel I aus einem ihrer funktionellen Derivate durch Behandeln mit einem solvolysierenden (z.B. hydrolysierenden) oder hydrogenolysierenden Mittel in Freiheit setzen.

So ist es möglich, nach einer der angegebenen Methoden eine Verbindung herzustellen, die der Formel I entspricht, aber an Stelle einer 5-Tetrazolylgruppe eine in 1-Stellung funktionell abgewandelte (durch eine Schutzgruppe geschützte) 5-Tetrazolylgruppe enthält. Als Schutzgruppen eignen sich beispielsweise: Triphenylmethyl, abspaltbar mit HCl oder Ameisensäure in einem inerten Lösungsmittel oder Lösungsmittelgemisch, z.B. Ether/ Dichlormethan/Methanol; 2-Cyanethyl, abspaltbar mit NaOH in Wasser/THF; p-Nitrobenzyl, abspaltbar mit $H_2$/Raney-Nickel in Ethanol (vgl. EP-A2-0 291 969).

Es ist ferner möglich, eine Verbindung der Formel I in eine andere Verbindung der Formel I umzuwandeln, indem man einen oder mehrere der Reste $R^1$, $R^2$ und/oder R in andere Reste $R^1$, $R^2$ und/oder R umwandelt, z.B. indem man Nitrogruppen (beispielsweise durch Hydrierung an Raney-Nickel in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Halogenatome (z.B. durch Reaktion mit Kupfer(I)cyanid) durch CN-Gruppen ersetzt und/oder Nitrilgruppen zu COOH-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder freie Hydroxy- und/oder NH-Gruppen mit einem unsubstituierten oder substituierten Alkyl- oder Ar-alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°. Von Bedeutung ist die Umwandlung eines Restes R, worin $R^4$ = H ist, in einen anderen Rest R, worin $R^4$ von H verschieden ist. Hier arbeitet man bevorzugt in einem Säureamid wie DMF, N-Methylpyrrolidon, 1,3-Dimethyl-2-oxo-hexahydropyrimidin oder Phosphorsäure-hexamethyl-triamid, einem Alkohol wie Methanol oder tert.-Butanol, einem Ether wie THF oder einem halogenierten Kohlenwasserstoff wie Dichlormethan oder Gemischen davon als Lösungsmittel und/oder in Gegenwart eines Alkalimetallalkoholats wie Natriummethylat oder Kalium-tert.-butylat, eines Alkalimetallhydrids wie Natrium- oder Kaliumhydrid, eines Alkalimetallcarbonats wie Natrium- oder Kaliumcarbonat, eines Alkalimetallbicarbonats wie Natrium- oder Kaliumbicarbonat oder eines tertiären Amins wie Triethylamin oder Ethyldiisopropylamin bei Temperaturen Zwischen etwa -30 und 200, vorzugsweise zwischen 20 und 60°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z. B. eine Verbindung der Formel I, die eine $NHCOCF_3$- oder eine $COOR^3$-Gruppe (worin $R^3$ von H verschieden ist) enthält, in die entsprechende Verbindung der Formel I umgewandelt werden, die stattdessen eine $NH_2$- oder eine HOOC-Gruppe enthält. Estergruppen können z.B. mit NaOH oder KOH in Wasser, Wasser-THF oder Wasser-Dioxan bei Temperaturen zwischen 0 und 100° verseift werden.

Umsetzung von Nitrilen der Formel I ($R^1$ und/oder $R^2$ = CN) mit Derivaten der Stickstoffwasserstoffsäure führt zu Tetrazolen der Formel I ($R^1$ und/oder $R^2$ = 5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vor zugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt vorzugsweise zwischen etwa 0,1 und 50 mg/kg, insbesondere 1 und 100 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "ubliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. MS (FAB) = Massenspektrum (erhalten nach der "fast atom bombardment"-Methode).

## Beispiel 1

Eine Lösung von 0,23 g Na in 20 ml Methanol wird innerhalb 15 Min. zugetropft zu einer Lösung von 3,2 g 2-Butyl-1(oder 3)H-imidazo[4,5-b]pyridin in 75 ml Methanol. Man rührt noch 30 Min. bei 20°, dampft ein, löst den Rückstand in 20 ml DMF und tropft bei 0° unter Rühren eine Lösung von 3,45 g 5-Brommethyl-2-(2-methoxycarbonylphenyl)-benzofuran in 10 ml DMF hinzu. Man rührt 16 Std. bei 20°,

dampft ein, arbeitet wie üblich auf, chromatographiert an Kieselgel und erhält 2-Butyl-3-(2-(2-methoxycarbonylphenyl)-benzofuran-5-yl-methyl)-3H-imidazo[4,5-b]pyridin.

**Beispiel 2**

(a) Man löst 1,89 g 2-Buty-7-methyl-3H-imidazo[4,5-b]pyridin (F. 75-76°) in 60 ml DMF, gibt bei -10 bis -14° 1,44 g Kaliumcarbonat hinzu, rührt 40 Min. bei -14° und tropft eine Lösung von 5,97 g 5-Brommethyl-2-(2-(1-triphenylmethyl-1H-tetrazol-5-yl)-phenyl)-benzofuran ("A") in 120 ml DMF hinzu. Das Gemisch wird dann noch 2 Std. bei -14° und 16 Std. bei 20° gerührt, eingedampft und mit Wasser/Ethylacetat aufgearbeitet. Nach Chromatographie zunächst mit Dichlormethan/Methanol 98:2, dann mit Toluol/Ethylacetat 8:2 erhält man 2-Butyl-7-methyl-3-(2-(2-(1-triphenylmethyl-1H-tetrazol-5-yl)-phenyl)-benzofuran-5-yl-methyl)-3H-imidazo[4,5-b]pyridin, Rf 0,61 (Dichlormethan/Methanol 97:3); MS (FAB) 706.

Das Ausgangsmaterial "A" wird wie folgt erhalten:
Bromierung von o-Tolunitril in Tetrachlormethan (UV-Bestrahlung) liefert o-Brommethyl-benzonitril (F. 69°). Dieses wird mit 5-Methylsalicylaldhyd in Aceton in Gegenwart von Kaliumcarbonat zu 2-(o-Cyanbenzyloxy)-5-methylbenzaldehyd (F. 99°) umgesetzt. Cyclisierung mit NaH in DMF unter Argon bei 20° gibt 2-o-Cyanphenyl-5-methyl-benzofuran (F. 112-113°), das mit Trimethylzinnazid in siedendem Toluol mit 2-o-(1-Trimethylstannyl-1H-tetrazol-5-yl)-phenyl-5-methyl-benzofuran [F. 289° (Zers.)] übergeführt wird. Abspaltung der Schutzgruppe mit HCl in Methanol/Ether führt zu 2-[2-(1H-Tetrazol-5-yl)-phenyl]-5-methyl-benzofuran, das in rohem Zustand mit Triphenylchlormethan in Dichlormethan in Gegenwart von Triethylamin in 2-[2-(1-Triphenylmethyl-1H-tetrazol-5-yl)-phenyl]-5-methyl-benzofuran (F. 167°) umgewandelt wird. Bromierung mit N-Bromsuccinimid gibt "A" (F. 169°).

(b) Das nach (a) erhaltene Produkt (Rf 0,61; 1 g) wird in 60 ml 4 n HCl in Dioxan gelöst und 16 Std. bei 20° gerührt. Nach Eindampfen und üblicher Aufarbeitung erhält man 2-Butyl-7-methyl-3-(2-(2-(1H-tetrazol-5yl)-phenyl)benzofuran-5-yl-methyl)-3H-imidazo[4,5-b]pyridin, F. 250° (Zers.); MS (FAB) 464.

Analog erhält man:

aus 2-Ethyl-5,7-dimethyl-3H-imidazo[4,5-b]pyridin und "A" das 2-Ethyl-5,7-dimethyl-3-(2-(2-(1-triphenylmethyl-1H-tetrazol-5-yl)-phenyl)-benzofuran-5-yl-methyl)-3H-imidazo[4,5-b]pyridin und daraus mit HCl/Dioxan das 2-Ethyl-5,7-dimethyl-3-(2-(2-(1H-tetrazol-5-yl)-phenyl)benzofuran-5-yl-methyl)-3H-imidazo-[4,5-b]pyridin, F. 256°;

aus 2-Butyl-5-(2-carboxybenzyl)-4,5-dihydro-4-oxo-3H-imidazo[4,5-c]pyridin ("C"; erhältlich durch Hydrogenolyse von 3-Benzyl-5-(2-benzyloxycarbonyl-benzyl)-2-butyl-4,5-dihydro-4-oxo-3H-imidazo[4,5-c]pyridin) und "A" das 2-Butyl-5-(2-carboxybenzyl)-4,5-dihydro-4-oxo-3-(2-(2-(1-triphenylme thyl-1H-tetrazol-5-yl)-phenyl)-benzofuran-5-yl-methyl)-3H-imidazo[4,5-c]pyridin und daraus mit HCl/Dioxan das 2-Butyl-5-(2-carboxybenzyl)-4,5-dihydro-4-oxo-3-(2-(2-(1H-tetrazol-5-yl)-phenyl)-benzofuran-5-yl-methyl)-3H-imidazo[4,5-c]-pyridin;

aus 2-Butyl-4,5-dihydro-4-oxo-3H-imidazo[4,5-c]pyridin und "A" das 2-Butyl-4,5-dihydro-4-oxo-3-(2-(2-(1-triphenylmethyl-1H-tetrazol-5-yl)-phenyl)-benzofuran-5-yl-methyl)-3H-imidazo[4,5-c]pyridin und daraus mit HCl/Dioxan das 2-Butyl-4,5-dihydro-4-oxo-3-(2-(2-(1H-tetrazol-5-yl)-phenyl)-benzofuran-5-yl-methyl)-3H-imidazo[4,5-c]pyridin;

aus 2-Butyl-5-carboxymethyl-4,5-dihydro-4-oxo-3H-imidazo[4,5-c]pyridin (erhältlich durch Hydrogenolyse von 3-Benzyl-5-benzyloxycarbonylmethyl-2-butyl-4,5-dihydro-4-oxo-3H-imidazo[4,5-c]pyridin) und "A" das 2-Butyl-5-carboxymethyl-4,5-dihydro-4-oxo-3-(2-(2-(1-triphenylmethyl-1H-tetrazol-5-yl)-phenyl)-benzofuran-5-yl-methyl)-3H-imidazo[4,5-c]pyridin und daraus mit HCl/ Dioxan das 2-Butyl-5-carboxymethyl-4,5-dihydro-4-oxo-3-(2-(2-(1H-tetrazol-5-yl)-phenyl)-benzofuran-5-yl-methyl)-3H-imidazo[4,5-c]pyridin.

**Beispiel 3**

Analog Beispiel 2a) erhält man mit 5-Brommethyl-2-(2-cyanphenyl)-benzofuran:
2-Butyl-3-(2-(2-cyanphenyl)-benzofuran-5-yl-methyl)-7-methyl-3H-imidazo[4,5-b]pyridin
3-(2-(2-Cyanphenyl)-benzofuran-5-yl-methyl)-2-ethyl-5,7-dimethyl-3H-imidazo[4,5-b]pyridin
2-Butyl-5-(2-carboxybenzyl)-3-(2-(2-cyanphenyl)-benzofuran-5-yl-methyl)-4,5-dihydro-4-oxo-3H-imidazo[4,5-c]pyridin
2-Butyl-3-(2-(2-cyanphenyl)-benzofuran-5-yl-methyl)-4,5-dihydro-4-oxo-3H-imidazo[4,5-c]pyridin
2-Butyl-5-carboxymethyl-3(2-(2-cyanphenyl)-benzofuran-5-yl)-methyl-4,5-dihydro-4-oxo-3H-imidazo[4,5-c]-pyridin.

**Beispiel 4**

Analog Beispiel 2 erhält man aus den dort angegebenen Ausgangsstoffen der Formel III mit 3-Brom-5-brommethyl-2-(2-(1-triphenylmethyl-1H-tetrazol-5-yl-phenyl)-benzofuran:
3-(3-Brom-2-(2-(1-triphenylmethyl-1H-tetrazol-5-yl)-phenylbenzofuran-5-yl-methyl)-2-butyl-7-methyl-3H-imidazo[4,5-b]pyridin und daraus 3-(3-Brom-2-(2-(1H-tetrazol-5-yl)-phenyl)-benzofuran-5yl-methyl)-2-butyl-7-methyl-3H-imidazo[4,5-b] pyridin; 3-(3-Brom-2-(2-(1-triphenylmethyl-1H-tetrazol-5-yl)-phenyl)-benzofuran-5-yl-methyl)-2-ethyl-5,7-dimethyl-3H-imidazo[4,5-b]pyridin und daraus 3-(3-Brom-2-(2-(1H-tetrazol-5-yl)-phenyl)-benzofuran-5-yl-methyl)-2-ethyl-5,7-dimethyl-3H-imidazo[4,5-b]pyridin;
3-(3-Brom-2-(2-(1-triphenylmethyl-1H-tetrazol-5-yl)-phenyl)-benzofuran-5-yl-methyl)-2-butyl-5-(2-carboxybenzyl)-4,5-dihydro-4-oxo-3H-imidazo[4,5-c]pyridin und daraus 3-(3-Brom-2-(2-(1H-tetrazol-5-yl)-phenyl)-benzofuran-5-yl-methyl)-2-butyl-5-(2-carboxybenzyl)-4,5-dihydro-4-oxo-3H-imidazo[4,5-c]pyridin;
3-(3-Brom-2-(2-(1-triphenylmethyl-1H-tetrazol-5-yl)-phenyl)-benzofuran-5-yl-methyl)-2-butyl-4,5-dihydro-4-oxo-3H-imidazo[4,5-c]-pyridin und daraus 3-(3-Brom-2-(2-(1H-tetrazol-5-yl)-phenyl-benzofuran-5-yl-methyl)-2-butyl-4,5-dihydro-4-oxo-3H-imidazo[4,5-c]pyridin;
3-(3-Brom-2-(2-(1-triphenylmethyl-1H-tetrazol-5-yl)-phenyl)-benzofuran-5-yl-methyl)-2-butyl-5-carboxymethyl-4,5-dihydro-4-oxo-3H-imidazo[4,5-c]pyridin und daraus 3-(3-Brom-2-(2-(1H-tetrazol-5-yl)-phenyl)-benzofuran-5-yl-methyl)-2-methyl-5-carboxymethyl-4,5-dihydro-4-oxo-3H-imidazo[4,5-c]pyridin.

**Beispiel 5**

Analog Beispiel 2a) erhält man aus 2-Butyl-1(oder3)H-imidazo [4,5-b]-pyridin: mit 5-Brommethyl-2-(2-nitrophenyl)-benzofuran das 2-Butyl-3-(2-(2-nitrophenyl)-benzofuran-5-yl-methyl)-3H-imidazo[4,5-b]pyridin, mit 5-Brommethyl-2-(2-trifluoracetamido-phenyl)-benzofuran das 2-Butyl-3-(2-(2-trifluoracetamido-phenyl)-benzofuran-5-yl-methyl)-3H-imidazo[4,5-b]pyridin;
mit 5-Brommethyl-2-(2-cyanphenyl)-3-fluor-benzofuran das 2-Butyl-3-(2-(2-cyanphenyl)-3-fluor-benzofuran-5-yl-methyl)-3H-imidazo[4,5-b]pyridin;
mit 5-Brommethyl-3-chlor-2-(2-ethoxycarbonyl-phenyl)-benzofuran das 2-Butyl-3-(3-chlor-2-(2-ethoxycarbonyl-phenyl)-benzofuran-5-yl-methyl)-3H-imidazo[4,5-b]pyridin.

**Beispiel 6**

Ein Gemisch von 1 g 2-Butyl-3-(2-(2-methoxycarbonylphenyl)-benzofuran-5-yl-methyl)-3H-imidazo[4,5-b]pyridin, 12 ml wässeriger 2 n NaOH-Lösung und 48 ml Ethanol wird 2 Std. gekocht, dann eingedampft. Nach Ansäuern mit HCl auf pH 3 erhält man 2-Butyl-3-(2-(2-carboxyphenyl)-benzofuran-5-yl-methyl)-3H-imidazo[4,5-b]pyridin, das abfiltriert, mit Wasser gewaschen und getrocknet wird.

**Beispiel 7**

Ein Gemisch von 420 mg 2-Butyl-3-(2-cyanphenyl)-benzofuran-5-yl-methyl)-7-methyl-3H-imidazo[4,5-b]-pyridin, 206 mg Trimethylzinnazid und 15 ml Xylol wird 96 Std. gekocht. Nach 48 Stunden gibt man nochmals 0,2 g Trimethylzinnazid hinzu. Man kühlt ab, versetzt mit etherischer Salzsäure und dampft ein. Nach üblicher Aufarbeitung erhält man 2-Butyl-7-methyl-3-(2-(2-(1H-tetrazol-5-yl)-phenyl)-benzofuran-5-yl-methyl)-3H-imidazo[4,5-b]pyridin, F. 250° (Zers.).

In üblicher Weise wird daraus das entsprechende K-Salz hergestellt.

**Beispiel 8**

Eine Lösung von 1 g 2-Butyl-3-(2-(2-nitrophenyl)-benzofuran-5-yl-methyl)-3H-imidazo[4,5-b]pyridin in 30 ml Ethanol wird an 1 g Raney-Ni bis zum Stillstand der $H_2$-Aufnahme bei 20° und Normaldruck hydriert. Man filtriert, dampft ein und erhält 3-(2-(2-Aminophenyl)-benzofuran-5-yl-methyl)-2-butyl-3H-imidazo[4,5-b]-pyridin.

**Beispiel 9**

Eine Lösung von 2,82 g Trifluormethansulfonsäureanhydrid in 10 ml Dichlormethan wird bei -50 bis -60° zugetropft zu einer Lösung von 3,96 g 3-(2-(2-Aminophenyl)-benzofuran-5-yl-methyl)-2-butyl-3H-imidazo[4,5-b]pyridin und 1,01 g Triethylamin in 30 ml Dichlormethan. Man läßt auf 20° erwärmen, gießt in

verdünnte Essigsäure und erhält nach üblicher Aufarbeitung 2-Butyl-3-(2-(2-trifluormethansulfonamido-phenyl)-benzofuran-5-yl-methyl)-3H-imidazo[4,5-b]pyridin.

**Beispiel 10**

Eine Lösung von 7,07 g 2-Butyl-3-(2-(2-(1-triphenylmethyl-1H-tetrazol-5-yl)-phenyl)-benzofuran-5-yl-methyl)-4,5-dihydro-4-oxo-3H-imidazo[4,5-c]pyridin und 1,17 g K-tert.-butylat in 25 ml DMF wird unter Rühren bei 20° tropfenweise mit einer Lösung von 0,79 g Chloracetonitril in 5 ml DMF versetzt. Man rührt noch 30 Min. bei 20°, gießt auf Eis, gibt Salzsäure bis pH 6 hinzu, arbeitet wie üblich auf und erhält 2-Butyl-5-cyanmethyl-3-(2-(2-(1-triphenylmethyl-1H-tetrazol-5-yl)-phenyl)-benzofuran-5-yl-methyl)-4,5-dihydro-4-oxo-3H-imidazo[4,5-c]pyridin.

Analog erhält man die folgenden 2-Butyl-3-(2-(2-(1-triphenylmethyl-1H-tetrazol-5-yl)-phenyl-benzofuran-5-yl-methyl)-4,5-dihydro-4-oxo-5-$R^4$-3H-imidazo[4,5-c]pyridine:
mit Methyliodid: 5-Methyl-
mit Ethyliodid: 5-Ethyl-
mit Isopropyliodid: 5-Isopropyl-
mit Butylbromid: 5-Butyl-
mit tert.-Butylbromid: 5-tert.-Butyl-
mit 3-Brompropionitril: 5-(2-Cyanethyl)-
mit 4-Brombutyronitril: 5-(3-Cyanpropyl)-
mit Bromessigsäuremethylester: 5-Methoxycarbonylmethyl-
mit 3-Brompropionsäureethyl-ester: 5-(2-Ethoxycarbonyl-ethyl)-
mit Benzylbromid: 5-Benzyl-
mit o-Fluorbenzylbromid: 5-(o-Fluorbenzyl)-
mit m-Fluorbenzylbromid: 5-(m-Fluorbenzyl)-
mit p-Fluorbenzylbromid: 5-(p-Fluorbenzyl)-
mit o-Chlorbenzylbromid: 5-(o-Chlorbenzyl)-
mit m-Chlorbenzylbromid: 5-(m-Chlorbenzyl)-
mit p-Chlorbenzylbromid: 5-(p-Chlorbenzyl)-
mit o-Brombenzylbromid: 5-(o-Brombenzyl)-
mit m-Brombenzylbromid: 5-(m-Brombenzyl)-
mit p-Brombenzylbromid: 5-(p-Brombenzyl)-
mit p-Methylbenzylbromid: 5-(p-Methylbenzyl)-
mit o-Trifluormethylbenzyl-bromid: 5-(o-Trifluormethylbenzyl)-
mit m-Trifluormethylbenzylbromid: 5-(m-Trifluormethylbenzyl)-
mit p-Trifluormethylbenzylbromid: 5-(p-Trifluormethylbenzyl)-
mit o-Methoxycarbonylbenzyl-bromid: 5-(o-Methoxycarbonylbenzyl)-
mit m-Methoxycarbonylbenzylbromid: 5-(m-Methoxycarbonylbenzyl)-
mit p-Methoxycarbonylbenzylbromid: 5-(p-Methoxycarbonylbenzyl)-
mit o-Cyanbenzylbromid: 5-(o-Cyanbenzyl)-
mit m-Cyanbenzylbromid: 5-(m-Cyanbenzyl)-
mit p-Cyanbenzylbromid: 5-(p-Cyanbenzyl)-
mit o-Nitrobenzylchlorid: 5-(o-Nitrobenzyl)-
mit m-Nitrobenzylchlorid: 5-(m-Nitrobenzyl)-
mit p-Nitrobenzylchlorid: 5-(p-Nitrobenzyl)-
mit o-Trifluoracetamidobenzylbromid: 5-(o-Trifluoracetamidobenzyl)-
mit m-Trifluoracetamidobenzylbromid: 5-(m-Trifluoracetamidobenzyl)-
mit p-Trifluoracetamidobenzylbromid: 5-(p-Trifluoracetamidobenzyl)-
mit o-Trifluormethylsulfonamidobenzylbromid: 5-(o-Trifluormethylsulfonamido-benzyl)-
mit m-Trifluormethylsulfonamidobenzylbromid: 5-(m-Trifluormethylsulfonamido-benzyl)-
mit p-Trifluormethylsulfonamidobenzylbromid: 5-(p-Trifluormethylsulfon-amido-benzyl)-.

**Beispiel 11**

Aus den in Beispiel 10 beschriebenen Verbindungen erhält man analog Beispiel 2(b) die nachstehenden 2-Butyl-4,5-dihydro-4-oxo-3-(2-(2-(1H-tetrazol-5-yl)-phenyl)-benzofuran-5-yl-methyl)-5-$R^4$-3H-imidazo[4,5-c]-pyridine:
5-Cyanmethyl-

5-Methyl-
5-Ethyl-
5-Isopropyl-
5-Butyl-
5-tert.-Butyl-
5-(2-Cyanethyl)-
5-(3-Cyanpropyl)-
5-Methoxycarbonylmethyl- (neben 5-Carboxymethyl-)
5-(2-Ethoxycarbonyl-ethyl)- [neben 5-(2-Carboxy-ethyl)-]
5-Benzyl-
5-(o-Fluorbenzyl)-
5-(m-Fluorbenzyl)-
5-(p-Fluorbenzyl)-
5-(o-Chlorbenzyl)-
5-(m-Chlorbenzyl)-
5-(p-Chlorbenzyl)-
5-(o-Brombenzyl)-
5-(m-Brombenzyl)-
5-(p-Brombenzyl)-
5-(p-Methylbenzyl)-
5-(o-Trifluormethyl-benzyl)-
5-(m-Trifluormethyl-benzyl)-
5-(p-Trifluormethyl-benzyl)-
5-(o-Methoxycarbonyl-benzyl)- [neben 5-(o-Carboxy-benzyl)-]
5-(m-Methoxycarbonyl-benzyl)- [neben 5-(m-Carboxy-benzyl)-]
5-(p-Methoxycarbonyl-benzyl)- [neben 5-(p-Carboxy-benzyl)-]
5-(o-Cyanbenzyl)-
5-(m-Cyanbenzyl)-
5-(p-Cyanbenzyl)-
5-(o-Nitrobenzyl)-
5-(m-Nitrobenzyl)-
5-(p-Nitrobenzyl)-
5-(o-Trifluoracetamido-benzyl)-
5-(m-Trifluoracetamido-benzyl)-
5-(p-Trifluoracetamido-benzyl)-
5-(o-Trifluormethylsulfonamido-benzyl)-
5-(m-Trifluormethylsulfonamido-benzyl)-
5-(p-Trifluormethylsulfonamido-benzyl)-.

## Beispiel 12

Analog Beispiel 10 erhält man durch Reaktion von 3-(3-Brom-2-(2-(1-triphenylmethyl-1H-tetrazol-5-yl)-phenyl)-benzofuran-5-yl-methyl)-2-butyl-4,5-dihydro-4-oxo-3H-imidazo[4,5-c]pyridin mit Chloracetonitril bzw. mit den anderen dort angegebenen Alkylierungsmitteln die nachstehenden 3-(3-Brom-2-(2-(1-triphenylmethyl-1H-tetrazol-5-yl)-phenyl)-benzofuran-5-yl-methyl)-2-butyl-4,5-dihydro-4-oxo-5-$R^4$-3H-imidazo[4,5-c]-pyridine:

5-Cyanmethyl-
5-Methyl-
5-Ethyl-
5-Isopropyl-
5-tert.-Butyl-
5-(2-Cyanethyl)-
5-(3-Cyanpropyl)-
5-Methoxycarbonylmethyl-
5-(2-Ethoxycarbonyl-ethyl)-
5-Benzyl-
5-(o-Fluorbenzyl)-
5-(m-Fluorbenzyl)-

5-(p-Fluorbenzyl)-
5-(o-Chlorbenzyl)-
5-(m-Chlorbenzyl)-
5-(p-Chlorbenzyl)-
5-(o-Brombenzyl)-
5-(m-Brombenzyl)-
5-(p-Brombenzyl)-
5-(p-Methylbenzyl)-
5-(o-Trifluormethyl-benzyl)-
5-(m-Trifluormethyl-benzyl)-
5-(p-Trifluormethyl-benzyl)-
5-(o-Methoxycarbonyl-benzyl)-
5-(m-Methoxycarbonyl-benzyl)-
5-(p-Methoxycarbonyl-benzyl)-
5-(o-Cyanbenzyl)-
5-(m-Cyanbenzyl)-
5-(p-Cyanbenzyl)-
5-(o-Nitrobenzyl)-
5-(m-Nitrobenzyl)-
5-(p-Nitrobenzyl)-
5-(o-Trifluoracetamido-benzyl)-
5-(m-Trifluoracetamido-benzyl)-
5-(p-Trifluoracetamido-benzyl)-
5-(o-Trifluormethylsulfonamido-benzyl)-
5-(m-Trifluormethylsulfonamido-benzyl)-
5-(p-Trifluormethylsulfonamido-benzyl)-

**Beispiel 13**

Analog Beispiel 2 (b) erhält man aus den in Beispiel 12 beschriebenen Verbindungen die nachstehenden 3-(3-Brom-2-(2-(1H-tetrazol-5-yl)-phenyl)-benzofuran-5-yl-methyl)-2-butyl-4,5-dihydro-4-oxo-5-$R^4$-3H-imidazo[4,5-c]pyridine:

5-Cyanmethyl-
5-Methyl-
5-Ethyl-
5-Isopropyl-
5-tert.-Butyl-
5-(2-Cyanethyl)-
5-(3-Cyanpropyl)-
5-Methoxycarbonylmethyl- (neben 5-Carboxymethyl-)
5-(2-Ethoxycarbonyl-ethyl)- [neben 5-(2-Carboxy-ethyl)-]
5-Benzyl-
5-(o-Fluorbenzyl)-
5-(m-Fluorbenzyl)-
5-(p-Fluorbenzyl)-
5-(o-Chlorbenzyl)-
5-(m-Chlorbenzyl)-
5-(p-Chlorbenzyl)-
5-(o-Brombenzyl)-
5-(m-Brombenzyl)-
5-(p-Brombenzyl)-
5-(p-Methylbenzyl)-
5-(o-Trifluormethyl-benzyl)-
5-(m-Trifluormethyl-benzyl)-
5-(p-Trifluormethyl-benzyl)-
5-(o-Methoxycarbonyl-benzyl)- [neben 5-(o-Carboxy-benzyl)-]
5-(m-Methoxycarbonyl-benzyl)- [neben 5-(m-Carboxy-benzyl)-]
5-(p-Methoxycarbonyl-benzyl)- [neben 5-(p-Carboxy-benzyl)-]

5-(o-Cyanbenzyl)-
5-(m-Cyanbenzyl)-
5-(p-Cyanbenzyl)-
5-(o-Nitrobenzyl)-
5-(m-Nitrobenzyl)-
5-(p-Nitrobenzyl)-
5-(o-Trifluoracetamido-benzyl)-
5-(m-Trifluoracetamido-benzyl)-
5-(p-Trifluoracetamido-benzyl)-
5-(o-Trifluormethylsulfonamido-benzyl)-
5-(m-Trifluormethylsulfonamido-benzyl)-
5-(p-Trifluornethylsulfonamido-benzyl)-.

**Beispiel 14**

Eine Lösung von 1 g 2-Butyl-4,5-dihydro-5-(o-nitrobenzyl)-4-oxo-3-(2-(2-(1H-tetrazol-5-yl)-phenyl)-benzofuran-5-yl-methyl)-3H-imidazo[4,5-c]pyridin in 20 ml Methanol wird an 0,3 g 5%ig Pd-Kohle bei 20° und Normaldruck bis zur Aufnahme der berechneten $H_2$-Menge hydriert. Man filtriert den Katalysator ab, dampft ein und erhält 5-(o-Aminobenzyl)-2-butyl-4,5-dihydro-4-oxo-3-(2-(2-(1H-tetrazol-5-yl)-phenyl)-benzofuran-5-yl-methyl)-3H-imidazo[4,5-c]pyridin.

Analog erhält man durch Hydrierung der entsprechenden in Beispiel 11 bzw. 13 genannten Nitroverbindungen die nachstehenden 2-Butyl-4,5-dihydro-4-oxo-3-(2-(2-(1H-tetrazol-5-yl)-phenyl)-benzofuran-5-yl-methyl)-3H-imidazo[4,5-c]pyridine:
5-(m-Aminobenzyl)-
5-(p-Aminobenzyl)-,
bzw. die nachstehenden 3-(3-Brom-2-(2-(1H-tetrazol-5-yl)-phenyl)-benzofuran-5-yl-methyl)-2-butyl-4,5-dihydro-4-oxo-4H-imidazo[4,5-c]-pyridine:
5-(o-Aminobenzyl)-
5-(m-Aminobenzyl)-
5-(p-Aminobenzyl)-.

Die nachstehenden Beispiele betreffen pharmazeutische Zubereitungen, die Wirkstoffe der Formel I oder ihre Salze enthalten.

**Beispiel A: Tabletten und Dragees**

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

**Beispiel B: Hartgelatine-Kapseln**

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

**Beispiel C: Weichgelatine-Kapseln**

Übliche Weichgelatine-Kapseln werden mit einem Gemisch aus jeweils 50 mg Wirkstoff und 250 mg Olivenöl gefüllt.

**Beispiel D: Ampullen**

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

**Beispiel E: Wässerige Suspension für orale Applikation**

Eine wässerige Suspension wird in üblicher Weise hergestellt. Die Einheitsdosis (5 ml) enthält 100 mg Wirkstoff, 100 mg Na-Carboxymethylcellulose, 5 ml Na-Benzoat und 100 mg Sorbit.

**Patentansprüche**

**1.** Benzofurane der Formel I

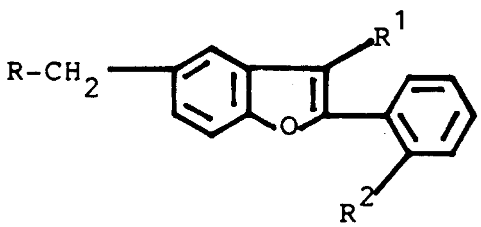

I

worin

R den Rest

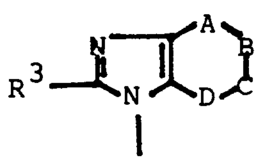

,

| | |
|---|---|
| $R^1$ | H, Hal, COOH, $CONH_2$, CHO, CN, $NH_2$ oder 5-Tetrazolyl, |
| $R^2$ | H, COOH, $COOR^3$, CN, $NO_2$, $NH_2$, $NHCOCF_3$, $NHSO_2CF_3$ oder 5-Tetrazolyl, |
| $R^3$ | H, Alkyl, Alkenyl oder Alkinyl mit jeweils bis zu 6 C-Atomen, |
| $R^4$ | H, Alkyl mit 1-6 C-Atomen, Cyan-alkyl, $R^3OOC$-alkyl, 5-Tetrazolyl-alkyl oder Ar-alkyl mit jeweils 1-6 C-Atomen im "alkyl"-Teil, |
| -A-B-C-D- | eine der Gruppen -CH=CH-CH=N, -CH=CH-N=CH-, -CH=N-CH=CH-, -N=CH-CH=CH-, -CH=CH-CO-$NR^4$-, -CH=CH-$NR^4$-CO-, -CO-$NR^4$-CH=CH- oder -$NR^4$-CO-CH=CH-, worin die H-Atome der -CH-Gruppen durch Alkyl mit 1-6 C-Atomen, Hal, $COOR^3$, CN und/oder 5-Tetrazolyl substituiert sein können, |
| Ar | unsubstituiertes oder ein- oder zweifach durch Hal, $R^3$, $CF_3$, $COOR^3$, CN, $OR^3$, $NO_2$, $NH_2$, $NH_2$, $NHCOCF_3$, $NHSO_2CF_3$ oder 5-Tetrazolyl substituiertes Phenyl und |
| Hal | F, Cl, Br oder I bedeuten, |

sowie ihre Salze.

**2.**

a) 2-Butyl-7-methyl-3-(2-(2-(1H-tetrazol-5-yl)-phenyl)-benzofuran-5-yl-methyl)-3H-imidazo[4,5-b]-pyridin.

b) 2-Ethyl-5,7-dimethyl-3-(2-(2-(1H-tetrazol-5-yl)-phenyl)-benzofuran-5-yl-methyl)-3H-imidazo [4,5-b]-pyridin.

c) 2-Butyl-5-(2-carboxybenzyl)-4,5-dihydro-4-oxo-3-(2-(2-(1H-tetrazol-5-yl)-phenyl)-benzofuran-5-yl-methyl)-3H-imidazo[4,5-c]pyridin.

d) 2-Butyl-4,5-dihydro-4-oxo-3-(2-(2-(1H-tetrazol-5-yl)-phenyl)-benzofuran-5-yl-methyl)-3H-imidazo-[4,5-c]pyridin.

e) 2-Butyl-5-carboxymethyl-4,5-dihydro-4-oxo-3-(2-(2-(1H-tetrazol-5-yl)-phenyl)-benzofuran-5-yl-methyl)-3H-imidazo[4,5-c]pyridin.

**3.** Verfahren zur Herstellung von Benzofuranen der Formel I nach Anspruch 1 sowie ihrer Salze, dadurch gekennzeichnet, daß man eine Verbindung der Formel II

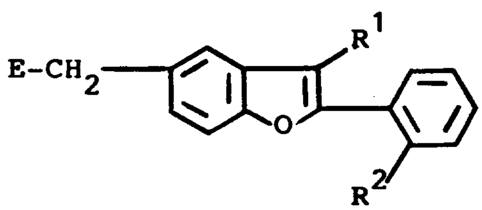

II

worin

E Cl, Br, I oder eine freie oder reaktionsfähig funktionell abgewandelte OH-Gruppe bedeutet und

$R^1$ und $R^2$ die in Anspruch 1 angegebene Bedeutung haben,

mit einer Verbindung der Formel III

H-R III

worin

R die in Anspruch 1 angegebene Bedeutung hat,

umsetzt,

oder daß man eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) $R^1$, $R^2$ und/oder R in einen oder mehrere andere Reste $R^1$, $R^2$ und/oder R umwandelt und/oder eine Base oder Säure der Formel I in eines ihrer Salze überführt.

**4.** Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- oder Hilfsstoff in eine geeignete Dosierungsform bringt.

**5.** Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

**6.** Verbindungen der Formel I nach Anspruch 1 und ihre physiologisch unbedenklichen Säureadditionssalze zur Bekämpfung von Krankheiten.

**7.** Verwendung von Verbindungen der Formel I nach Anspruch 1 und/oder ihrer physiologisch unbedenklichen Säureadditionssalze zur Herstellung eines Arzneimittels.

**8.** Verwendung von Verbindungen der Formel I nach Anspruch 1 und/oder ihrer physiologisch unbedenklichen Säureadditionssalze bei der Bekämpfung von Krankheiten.